# EUROPEAN PATENT SPECIFICATION

(11) **EP 2 701 626 B1**
(45) Date of publication and mention of the grant of the patent: **16.08.2017**
(21) Application number: 12720675.3
(22) Date of filing: 26.04.2012
(51) Int. Cl.: A61B 34/30, A61B 90/70

(54) **OPERATING DEVICE FOR OPERATING A MEDICAL INSTRUMENT IN A CLEANING AND DISINFECTION MACHINE**
BEDIENUNGSVORRICHTUNG FÜR DEN BETRIEB EINES MEDIZINISCHEN INSTRUMENTS IN EINEM REINIGUNGS-UND DESINFEKTIONSAUTOMAT
DISPOSITIF D'ACTIONNEMENT DESTINÉ À ACTIONNER UN INSTRUMENT MÉDICAL DANS UNE MACHINE DE NETTOYAGE ET DE DÉSINFECTION

(30) Priority: 29.04.2011 NL 2006689
(43) Date of publication of application: 05.03.2014
(73) Proprietor: P.M.T. Partners Medische Techniek B.V., 2952 BD Alblasserdam (NL)
(72) Inventor: SCHOUWENBURG, John, NL-3356 MR Papendrecht (NL); SCHOUWENBURG, Marco, NL-3514 VW Utrecht (NL)
(74) Representative: Algemeen Octrooi- en Merkenbureau B.V.
(86) International application number: PCT/NL2012/050277
(87) International publication number: WO 2012/148266

(56) References cited:
- DE-A1- 10 248 460
- US-A- 5 554 228
- US-A1- 2006 048 787
- US-A1- 2009 158 539
- US-A1- 2010 298 843

## Description

The invention relates to an operating device for operating a medical instrument in a cleaning and disinfection machine.

It is known to clean a medical instrument provided with one or more moveable instrument parts, for instance an intervention instrument for use with an endoscopic procedure in the human body, such as the Intuitive Surgical EndoWrist ®, manually after use. Therein the disinfection worker operates operating elements mounted on the medical instrument, in such a way that during the cleaning process the one or more moveable instrument parts are moved, so that the water can reach the various instrument parts well.

A disadvantage however is that, because of the disinfection worker manually operating and cleaning the medical instrument, the final result leaves a lot to be desired. It occurs, for example, that on the contact surfaces of moving parts of the instrument dirt stays behind, which at a subsequent use of the instrument gives risk of infection.

Another disadvantage is that the disinfection worker may become tired after some time, as a result of which the final result can also be influenced in a negative way.

Document US 5554228 A discloses a device for cleaning surgical instruments whereby an operating device drives activation members, which are engaged to handles of surgical instruments, in a simultaneous manner. Therefore it is an object of the invention to provide a device that achieves a cleaning and disinfection result, wherein differences in quality regarding the final result of the disinfection process are minimized.

It is a further object of the invention to provide a device, wherein possible tiredness of the disinfection worker has no or a negligible impact on the final result of the cleaning and disinfection process.

The invention is described in claims 1 and 10. Preferred embodiments are described in the dependent claims. Hereto the operating device according to the invention is provided with:
- a mounting part for mounting a medical instrument, wherein the instrument is provided with an operating element with which a moveable instrument part can be moved,
- an activation member, during use provided near the operating element, such as on the mounting part, facing towards the instrument and adapted to engage the operating element,
- wherein the activation member during use is driveably connected to a drive,
- in such a way that the moveable instrument part is moveable by the drive.

Because the operating device is provided with a driveable activation member, that engages the operating element of the instrument, the cleaning and disinfection process can be automated. The activation member can for that matter be provided advantageously on or near the mounting part. Therein the operating device is arranged along with the medical instrument in a disinfection space, such as a washing cabin, of a disinfection machine, wherein the instrument is disinfected by means of a disinfection fluid. During the disinfection process the drive connected to the activation member is activated, as a result of which the moveable instrument part moves. Thus it is achieved that the disinfection fluid can reach the moveable instrument part and further instrument parts well, which provides that a relatively optimal final result of the cleaning and disinfection process is achieved. Furthermore, differences in quality regarding the final result of the disinfection process are minimized, as a result of which the final result becomes reproducible.

In an embodiment the activation member is arranged rotatably on a shaft, wherein the rotatable activation member is arranged for engaging, during use, a rotatable operating element of the instrument, wherein the axes of rotation of the activation member and the operating element are substantially aligned. Because relatively many medical instruments are equipped with moveable instrument parts with rotatable operating elements, it is advantageous to embody the activation member as being rotatable as well, wherein the axes of rotation are aligned. Thus, a relatively firm connection with such a rotatable operating element is achieved, which promotes operability of the instrument during disinfection.

In a further embodiment the rotatable activation member comprises a recess arranged for receiving a cam arranged on the rotatable operating element during use. Since operating elements of such a medical instrument are often provided with a cam for precise positioning of a moveable instrument part, it is advantageous to provide the rotatable activation member with one or more recesses that fit the cam. The operating device according to the invention is provided with two or more activation members arranged in a row, wherein the operating device comprises activation means driveable by the drive near the row of activation members, which means are arranged for letting the activation members be driveable by the drive in a non-simultaneous manner. It should be prevented that during cleaning multiple operating elements are activated simultaneously, as a result of which the device could be damaged. This can be achieved for example in a mechanical way, but also in an electronic way, wherein for example servo motors connected to the activation members are activated individually by an electronic operating system. The driveable activation means can also be used to drive just one activation member. The risk of simultaneously activation of multiple activation members, of course, is not present then.

In yet a further embodiment the activation members arranged in a row are arranged rotatably on shafts, wherein the rotatable activation members are arranged, during use, for engaging rotatable operating elements of the instrument, wherein the axes of rotation are substantially aligned, and each shaft is provided with a sprocket wheel. Such a mechanical construction with a sprocket wheel allows various forms of driving and at the same time offers good reliability, especially during use in a moist environment, such as the washing cabin of a disinfection machine.

In yet a further embodiment the activation means comprise an element that is displaceable in a longitudinal direction with respect to the row of sprocket wheels, on which element an activation pin facing the sprocket wheels is arranged, in such a way that when moving the activation pin along the sprocket wheels, it engages the sprocket wheels in a non-simultaneous manner. In fact the sprocket wheels are activated sequentially in this way by the displaceable element with the activation pin moving past the sprocket wheels, as a result of which simultaneous operation of the activation members and operating elements connected to the sprocket wheels is prevented. Preferably, the movement of the displaceable element concerns a reciprocating movement past the sprocket wheels, and the drive is arranged for causing such a movement.

It is advantageous to mount multiple activation pins grouped in a row on the displaceable element in longitudinal direction. The amount of activation pins therein can be adjusted. By mounting a greater amount of activation pins in a row in a longitudinal direction of the element a greater rotation of the sprocket wheels is achieved, because each activation pin is arranged for being received between two sprockets of a sprocket wheel.

In a further embodiment the activation means comprise an element with two or more activation pins facing the respective sprocket wheels which element is displaceable perpendicular to the direction of the row of activation members, wherein the activation pins are arranged on the displaceable element in the displacement direction at such a distance from each other that when moving the activation pins past the sprocket wheels these pens engage the sprocket wheels in a non-simultaneous manner. It is also possible that a displaceable element is displaceable in a direction perpendicular to the direction of the activation members. To prevent that the activation members are moved simultaneously the activation pins are spaced apart in the displacement direction. Of course it is also possible to use a combination of displacement directions.

In a yet further embodiment a slip coupling is arranged between the shaft and the sprocket wheel. The slip coupling prevents that the activation member connected to the shaft at an extreme position of the operating element corresponding to the activating member is activated even more, to prevent that the instrument is damaged. Preferably, the slip coupling concerns a so-called 'multi-wave' spring.

In a yet even further embodiment the displaceable element is provided with a rack arranged parallel to the longitudinal direction of the displaceable element, that during use engages a sprocket wheel connected to the drive. Such a construction is relatively reliable in a moist environment, such as a disinfection space. Additionally the rack can be connected easily to the sprocket wheel of the drive or decoupled from the sprocket wheel, such that the operating device can be removed from the machine.

In a yet further embodiment the operating device is provided with a supply duct that is arranged for supplying, during use, disinfection fluid into an opening in the instrument. In order to fully clean and disinfect the internal parts of the instrument as well, such as cables running through an instrument arm from the operating elements towards the moveable instrument part, it is advantageous to provide the device with one or more supply ducts for disinfection fluid, wherein a supply duct is connected to an opening in the instrument during use. After disinfection the supply duct can also be used to supply medical compressed air through the opening into the instrument in order to dry the internal parts.

Furthermore, it is advantageous to connect the supply duct to a pressure gauge in order to check whether the internal disinfection progresses satisfactorily: at a too low flushing pressure leakage may be occurring, as a result of which the cleaning and disinfection result can be at risk, at a too high flushing pressure on the contrary it could be the case that a blockage has occurred in the internal parts of the instrument, because of which the cleaning and disinfection result can be at risk. The pressure gauge can be in communicative connection with an alarm that goes off when such a wrong pressure is detected.

The invention furthermore relates to a cleaning and disinfection machine apparently arranged for use of a operating device according to the invention.

In an embodiment the cleaning and disinfection machine is provided with a disinfection space, an input part connected to a side of the disinfection space for supplying an operating device according to the invention into the disinfection space by means of a transportation system arranged in the machine, an output part connected to another side of the disinfection space for removing the operating device from the disinfection space by the transportation system, wherein on or near a wall of the disinfection space a drive is arranged for driving, during use, the operating device, that is detachably connected to the drive, wherein the disinfection space during use of the input part as well as the output part is closeable by means of a moveable closing member.

In a further embodiment the machine is provided with a detection system, that is arranged for detecting the result of the disinfection. In order to ensure an even more constant quality of the disinfection result, the cleaning and disinfection machine can be equipped with such a system in order to inform the operator of the machine, or a control system connected to the disinfection machine, about the achieved result of disinfection.

In a yet further embodiment the detection system is in communicative connection with a control system that is arranged for letting the closing member open at a desired result of disinfection and for letting the operating device be removed by the transportation system to the output part, and for keeping the closing member closed at an undesired result of disinfection and to carry out a further disinfection. In this way an even higher and more constant quality is achieved, because at an undesired disinfection result another disinfection would be carried out, until the desired result is achieved.

### Detailed description

An exemplary embodiment of a cleaning and disinfection machine with an operating device according to the invention, as well as an operating device according to the invention will be elucidated by way of example with reference to the accompanying figures.
Fig. la shows an embodiment of a cleaning and disinfection machine with an operating device according to the invention in side view,
Fig. 1b shows an embodiment of a cleaning and disinfection machine with an operating device according to the invention in top view,
Fig. 2 shows an embodiment of an operating device according to the invention in top view and
Fig. 3 shows an embodiment of an operating device according to the invention in side view.

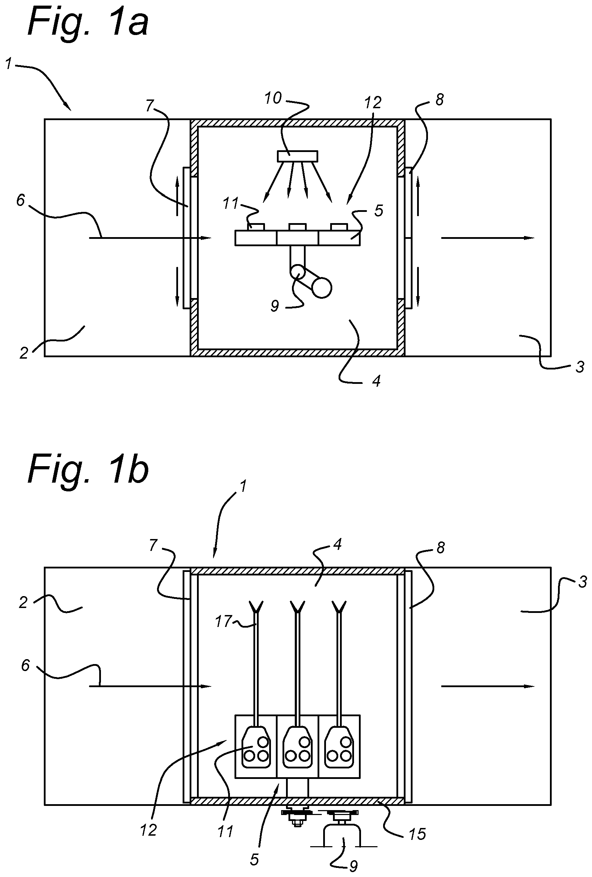

Figure 1a shows a cleaning and disinfection machine 1 provided with a washing cabin 4 arranged in the center of the cleaning and washing machine 1. The washing cabin 4 is delimited by the walls of the cleaning and disinfection machine 1 and two doors 7, 8. In the washing cabin 4 the operating device 12 according to the invention is arranged. According to the embodiment shown comprises among others three adjacent mounting blocks 5, wherein on each block 5 a medical instrument 11 is arranged for being disinfected. Another number of mounting blocks 5 is also conceivable. The operating device 12 is detachably connected to a drive 9 arranged at the outside of the washing cabin 4. The cleaning and disinfection machine 1 is furthermore provided with a transportation system 6, such as a roller belt, for moving the operating device 12 through the cleaning and disinfection machine 1.

At the start of the disinfection cycle the first door 7 opens in order to allow an operating device 12 with dirty instruments 11 into the washing cabin 4. The second door 8 is closed at this moment. The space comprised by the side walls of the cleaning and disinfection machine and the closed door 8 is indicated as input part 2. At transportation of the operating device 12 into the washing cabin 4 by the transportation system 6 a connection is established between the drive 9 and the operating device 12. Subsequently, the first door 7 is closed in order to start the disinfection. The disinfection is now started by the injection of water by one or more water sprayers 10 into the washing cabin 4. During the disinfection moveable parts of the medical instrument 11 are moved in such a way that the water can fully reach the different instrument parts. This movement occurs automatically by activation of the drive 9 that is connected to one or more operating elements on the medical instrument 11. If desired internal parts of an instrument 11 can be disinfected by connecting these parts to one or more water ducts arranged in the washing cabin 4, which ducts supply water under pressure to the internal parts of the instrument 11. When the disinfection is completed, the second door 8 opens and the operating device 12 with the disinfected instrument 11 is transported through an output part 3 comprised by the walls of the machine 1 and the closed first door 7, and removed from the machine 1. Additionally it is possible by way of example to implement a checking mechanism in the machine 1, that checks whether the disinfection is carried out correctly. The checking mechanism therein is in communicative connection with the second door 8. When a correct disinfection has been carried out, the second door 8 opens and the operating device 12 is removed from the machine 1, when an incorrect disinfection has been carried out the second door 8 remains closed and another disinfection can be carried out in the washing 4 until a correct disinfection is achieved.

Figure 1b show the same cleaning and disinfection machine 1, but now in plan view. In this figure the medical instruments 11 can be seen that comprise elongated arms 17. In the washing cabin the arms 17 are directed away from the washing cabin wall, such that the water can fully reach the various instrument parts.

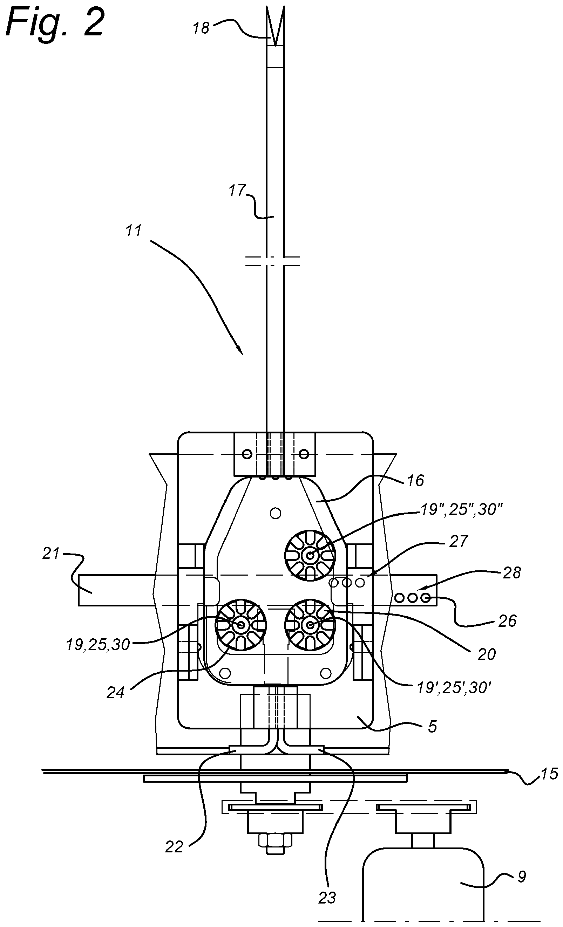

Figure 2 shows an operating device 12 according to the invention in plan view. A medical instrument 11 therein is arranged on a mounting block 5. The instrument 11 is provided with an elongated arm 17, with a gripper 18 at the end of the arm 17. One or more cables (not shown) run through the arm 17, which cables are each connected to an operating wheel 24 on an instrument housing 16. Each operating wheel 24 is arranged, during use, on a drive disc 19, 19', 19". The medical instrument 11 is provided with three operating wheels 24, but another number of wheels is conceivable; this depends of the type of instrument that needs to be cleaned and disinfected. The drive discs 19, 19', 19" are each connected to a part of the gripper 18 by means of the operating wheels 24 via one of the mentioned cables in such a way that the gripper 18 at a rotation of a drive disc 19, 19' and 19" is moved over one degree of freedom. A degree of freedom can be thought of as opening and closing of the gripper 18, rotating the gripper 18 around a longitudinal axis, or rotating the gripper 18 around a transversal axis. The number of degrees of freedom depends among others on the type of instrument 11 that is arranged on the mounting block 5.

The mounting block 5 is arranged on a frame that is produced in such a way that multiple mounting blocks 5 can be arranged next to each other in a modular fashion, in such a way that multiple instruments 11 can be cleaned at the same time. In order to clean the housing 16, the elongated arm 17 and the gripper 18 internally, the housing 16 is provided with openings (not shown) arranged at the side of the mounting wall 15 to which flushing ducts 22, 23 are connected. Water can be injected under pressure into the internal parts of the housing 16 via the flushing ducts 22, 23, wherein the water flows into the instrument arm 17 and the gripper 18 past the mentioned cables because of the flushing pressure, as a result of which an even better cleaning and disinfection is achieved. The flushing ducts 22, 23 can be connected to a pressure gauge for checking whether the internal disinfection progresses as desired: at a too low flushing pressure leakage may be present, as a result of which the cleaning and disinfection result can be at risk, at a too high flushing pressure on the contrary a blockage may have occurred in the internal parts of the instrument 11, as a result of which the cleaning and disinfection result can be at risk as well. The pressure gauge therein can be in communicative connection with an alarm that goes off when such an incorrect pressure has been found. Additionally, after disinfection the flushing ducts 22, 23 can be connected to medical compressed air to blow the internal parts of the instrument 11 dry.

Each drive disc 19, 19', 19" as shown is provided with eight recesses 20. These recesses 20 are intended for receiving a cam that may be arranged on an operating wheel 24.

The drive discs 19, 19', and 19" furthermore are each connected to shafts 25, 25', 25", running away from the reader in viewing direction, arranged in the mounting block 5. The shafts 25, 25', 25" are each provided with sprocket wheels 30, 30', 30" near the underside of the mounting block 5. At rotation of the sprocket wheels 30, 30', 30" the respective drive disc 19, 19', 19" is rotated. Also for this purpose a drive beam 21 is arranged below the mounting block 5, provided with two sets of activation pins 27, 28 with three activation pins 26 each. The drive beam 21 is connected to the drive 9. When moving the drive beam 21 to the left the first set of activation pins 27 moves past the sprocket wheel 30" connected to drive disc 19", which causes a rotation of the drive disc 19" and the operating wheel 24 connected to the drive disc 19". Subsequently the second set of activation pins 28 consecutively moves past the sprocket wheels 30 and 30', as a result of which the activation members 19, 19' are moved.

The two sets of activation pins 27, 28 are spaced apart, in transversal direction as well as in longitudinal direction of the drive beam 21. Thus it is achieved that the drive discs 19, 19', 19" are moved consecutively, wherein drive disc 19" is moved first, then drive disc 19' and lastly drive disc 19. The consecutive or sequential movement of the drive discs 19, 19', 19" prevents that the drive discs 19, 19', 19" and the operating elements or -wheels 24 connected thereto are operated at the same time, as a result of which the instrument 11 otherwise would be broken. Also when moving back the drive beam 21 to the right the drive discs 19, 19', 19" are moved consecutively, although in reversed order.

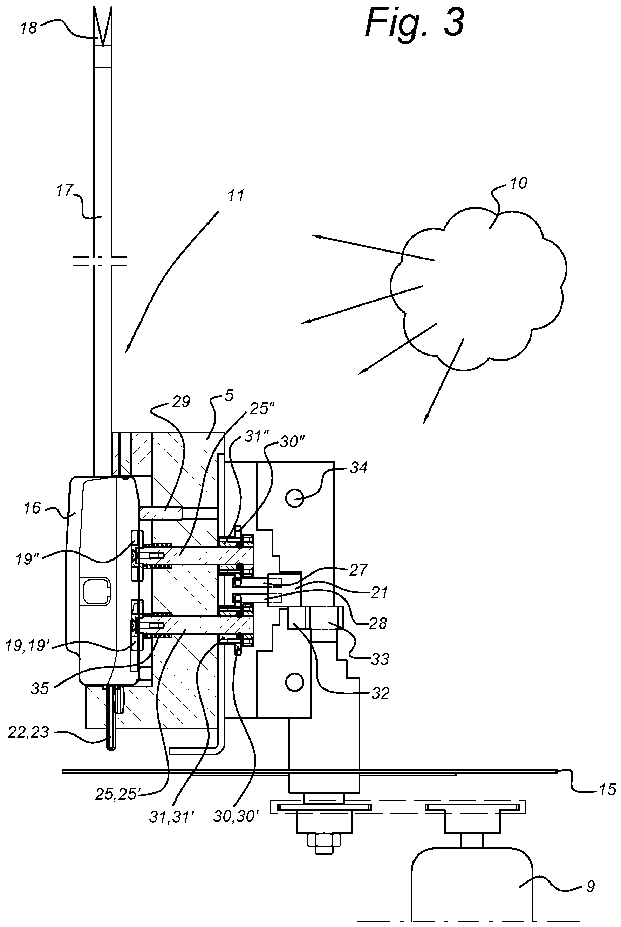

Figure 3 shows a side view of an operating device 12 according to the invention, wherein the mounting block 5 is partly shown in cross-section. In figure 3, beside what has been discussed with respect to figure 2, an adjustment pin 29 can be seen, which is arranged in the mounting block 5 and supports the instrument 11. The adjustment pin 29 serves to adjust the space present between the instrument housing 16 and the mounting block 5. This space serves to also allow water below the instrument housing 16, as a result of which the instrument will be disinfected sufficiently at that location. It can also be seen that the rotation surfaces of the drive discs 19, 19' and 19" are spaced at a certain distance from the mounting block 5. Around the shafts 25, 25', 25" a spring 35 is arranged below the rotation surfaces in order to obtain a good connection between the drive discs 19, 19', 19" and the respective operating elements 24 on the instrument 11. Additionally it can be seen that near the underside of the shafts 25, 25', 25" sprocket wheels 30, 30', 30" are arranged. Between each sprocket wheel 30, 30', 30" and the corresponding shaft 25, 25', 25" a slip coupling 31, 31', 31" is arranged, which breaks the connection between the sprocket wheel 30, 30', 30" and the shaft 25, 25', 25" when an operating wheel 24 connected to a drive disc 19, 19', 19" has reached an extreme position. Near each slip coupling 31, 31', 31" an O-ring is arranged on the shaft 25, 25', 25" to configure the slip coupling 31, 31', 31".

Also the drive beam 21 can be seen, which extends in the viewing direction. The sets of activation pins 27, 28 are arranged on the drive beam 21, which pens touch and rotate the sprocket wheels 30, 30', 30" when moving the drive beam 21 in the viewing direction.

The drive beam 21 furthermore is fixedly connected with one or more support parts 36, wherein the support parts 36 serve to support the drive beam 21. The support parts 36 are arranged at regular intervals along the length of the drive beam 21 and furthermore can slide over guide bars 34 arranged below the mounting block 5. These guide bars 24 serve to allow the drive beam 21 to make a smooth translating movement in the viewing direction.

Additionally, on the drive beam 21, near the underside, a rack 32 is arranged that engages a sprocket wheel 33. The sprocket wheel 33 furthermore is driveably connected to a drive 9.

### Reference numerals

- 1.: Cleaning- and disinfection machine
- 2.: Input part
- 3.: Output part
- 4.: Washing cabin
- 5.: Mounting block
- 6.: Transportation system
- 7.: First door
- 8.: Second door
- 9.: Block drive
- 10.: Water sprayer
- 11.: Medical instrument
- 12.: Operating device
- 13.: -
- 14.: -
- 15.: Connecting wall
- 16.: Instrument housing
- 17.: Instrument arm
- 18.: Gripper
- 19.: Drive disc (19', 19")
- 20.: Recess in drive disc
- 21.: Drive beam
- 22.: First flushing channel
- 23.: Second flushing channel
- 24.: Operating wheel
- 25.: Shaft (25', 25")
- 26.: Activation pin
- 27.: First set of activation pins
- 28.: Second set of activation pins
- 29.: Adjustment pin
- 30.: Sprocket wheel (30', 30")
- 31.: Slip coupling (31', 31")
- 32.: Rack
- 33.: Sprocket wheel
- 34.: Guide bar
- 35.: Spring
- 36.: Support part

## Claims

1. Operating device (12) for operating a medical instrument (11) in a cleaning and disinfection machine (1), comprising:
- a mounting part (5) for mounting a medical instrument (11), wherein the instrument (11) is provided with an operating element (24) with which a moveable instrument part (18) can be moved,
- an activation member (19; 19'; 19"), during use provided near the operating element (24), such as on the mounting part (5), facing towards the instrument (11) and adapted to engage the operating element (24),
- wherein the activation member (19; 19'; 19") during use is driveably connected to a drive (9),
- in such a way that the moveable instrument part (18) is moveable by the drive (9),
- wherein two or more activation members are provided, arranged in a row (19, 19'; 19', 19"), wherein the operating device (12) comprises activation means driveable by the drive (9) near the row of activation members (19, 19'; 19', 19"), which means are arranged for letting the activation members (19, 19', 19") be driveable by the drive (9) in a non-simultaneous manner.

2. Operating device (12) according to claim 1, wherein a rotatable activation member (19; 19'; 19") comprises a recess (20) arranged for receiving a cam arranged on the rotatable operating element (24) during use.

3. Operating device (12) according to claim 1 or 2, wherein the activation members arranged in a row (19, 19'; 19', 19") are arranged rotatably on shafts (25, 25', 25"), wherein the rotatable activation members (19, 19', 19") are arranged for, during use, engaging rotatable operating elements (24) of the instrument, wherein the axes of rotation are substantially aligned, and each shaft (25; 25'; 25") is provided with a sprocket wheel (30; 30'; 30"), that is driveable by the activation means.

4. Operating device (12) according to claim 3, wherein the activation means comprise an element (21) that is displaceable in a longitudinal direction with respect to the row of sprocket wheels (30, 30'), on which element an activation pin (26, 28) facing the sprocket wheels (30, 30') is arranged, in such a way that when moving the activation pin (26, 28) past the sprocket wheels (30, 30'), it engages the sprocket wheels (30, 30') in a non-simultaneous manner.

5. Operating device (12) according to claim 3, wherein the activation means comprise an element (21) with two or more activation pins (26, 27, 28) facing the respective sprocket wheels (30', 30") which element (21) is displaceable perpendicular to the direction of the row of activation members (19', 19"), wherein the activation pins are arranged on the displaceable element in the direction of displacement at such a distance from each other that when moving the activation pins (26, 27, 28) past the sprocket wheels (30', 30") these pins engage the sprocket wheels (30', 30") in a non-simultaneous manner.

6. Operating device (12) according to one of the claims 3-5, wherein a slip coupling (31; 31'; 31") is arranged between the shaft (25; 25'; 25") and the sprocket wheel (30; 30'; 30").

7. Operating device (12) according to any one of the claims 4-6, wherein the displaceable element (21) is provided with a rack (32) arranged parallel to the longitudinal direction of the displaceable element (21), that during use engages a sprocket wheel (33) connected to the drive (9).

8. Operating device (12) according to one of the preceding claims, wherein the operating device (12) is provided with a supply duct (22; 23) that is arranged for supplying, during use, disinfection fluid into an opening in the instrument (11).

9. Operating device (12) according to claim 8, wherein the supply duct (22; 23) is connected to a pressure gauge.

10. Cleaning and disinfection machine (1) provided with a disinfection space (4), an input part connected to a side of the disinfection space (4) for supplying an operating device (12) according to one of the claims 1-9 into the disinfection space (4) by means of a transportation system (6) arranged in the machine (1), an output part (3) connected to another side of the disinfection space (4) for removing the operating device (12) from the disinfection space (4) by the transportation system (6), wherein on or near a wall (15) of the disinfection space (4) a drive is arranged for driving, during use, the operating device (12), that is detachably connected to the drive (9), wherein the disinfection space (4) during use of the input part (2) as well as the output part (3) is closeable by means of a moveable closing member (7, 8).

11. Cleaning and disinfection machine (1) according to claim 10, provided with a detection system, that is arranged for detecting the disinfection result.

12. Cleaning and disinfection machine (1) according to claim 11, wherein the detection system is in communicative connection with a control system that is arranged for allowing the closing member (8) to open at a desired disinfection result and for allowing the operating device (12) to be removed by the transportation system (6) to the output part (3), and for keeping the closing member (8) closed at an undesired disinfection result and carrying out a further disinfection.

## Patentansprüche

1. Bedienungsvorrichtung (12) für den Betrieb eines medizinischen Instruments (11) in einer Reinigungs- und Desinfektionsmaschine (1), umfassend
- ein Montageteil (5) zur Montage eines medizinischen Instruments (11), worin das Instrument (11) mit einem Bedienelement (24) versehen ist, mit dem ein beweglicher Instrumententeil (18) bewegt werden kann,
- ein Aktivierungsglied (19, 19', 19"), das während des Gebrauchs in der Nähe des Bedienelements (24), wie auf dem Montageteil (5), vorgesehen ist, dem Instrument (11) zugewandt ist und angepasst ist, um das Bedienelement (24) zu betätigen,
- worin das Aktivierungsglied (19. 19', 19") während des Gebrauchs antreibbar mit einem Antrieb (9) verbunden ist,
- sodass der bewegliche Instrumententeil (18) durch den Antrieb (9) bewegbar ist,
- worin zwei oder mehr Aktivierungsglieder (19, 19', 19") vorgesehen sind, die in einer Reihe (19, 19'; 19', 19") angeordnet sind, worin die Bedienungsvorrichtung (12) durch den Antrieb (9) antreibbare Aktivierungsmittel in der Nähe der Reihe (19, 19'; 19', 19") der Aktivierungsglieder (19, 19', 19") umfasst, die so angeordnet sind, dass die Aktivierungsglieder (19, 19', 19") durch den Antrieb (9) in einer nicht simultanen Weise antreibbar sind.

2. Bedienungsvorrichtung (12) nach Anspruch 1, worin ein drehbares Aktivierungsglied (19, 19', 19") eine Aussparung (20) umfasst, die angeordnet ist, um eine Nocke während des Gebrauchs aufzunehmen, die auf dem drehbaren Bedienelement angeordnet ist.

3. Bedienungsvorrichtung (12) nach Anspruch 1 oder 2, worin die in einer Reihe (19, 19'; 19', 19") angeordneten Aktivierungsglieder (19, 19', 19") auf drehbaren Schäften (25, 25', 25") angeordnet sind, worin die drehbaren Aktivierungsglieder (19, 19', 19") angeordnet sind, um, während des Gebrauchs, die drehbaren Bedienelemente (24) des Instruments zu betätigen, worin die Rotationsachsen im Wesentlichen ausgerichtet sind und jeder Schaft (25, 25', 25") mit einem Kettenrad (30, 30', 30") versehen ist, das durch die Aktivierungsmittel antreibbar ist.

4. Bedienungsvorrichtung (12) nach Anspruch 3, worin die Aktivierungsmittel ein Element (21) umfassen, das in Längsrichtung in Bezug auf die Reihe von Kettenrädern (30, 30') verschiebbar ist, wobei an dem Element ein den Kettenrädern (30, 30') zugewandter Aktivierungsstift (26, 28) angeordnet ist, sodass bei Bewegung des Aktivierungsstifts (26, 28) vorbei an den Kettenrädern (30, 30'), dieser die Kettenräder (30, 30') in einer nicht simultanen Weise betätigt.

5. Bedienungsvorrichtung (12) nach Anspruch 3, worin die Aktivierungsmittel ein Element (21) mit zwei oder mehr den Kettenrädern (30', 30") zugewandten Aktivierungsstiften (26, 27, 28) umfassen, wobei das Element (21) senkrecht zur Richtung der Reihe von Aktivierungsgliedern (19,', 19") verschiebbar ist, wobei die Aktivierungsstifte auf dem verschiebbaren Element in Richtung der Verschiebung in einem derartigen Abstand voneinander angeordnet sind, dass bei Bewegung der Aktivierungsstifte (26, 27, 28) vorbei an den Kettenrädern (30', 30") diese Stifte die Kettenräder (30', 30") in einer nicht simultanen Weise betätigen.

6. Bedienungsvorrichtung (12) nach einem der Ansprüche 3-5, worin eine Schlupfkupplung (31, 31', 31") zwischen dem Schaft (25, 25', 25") und dem Kettenrad (30, 30', 30") angeordnet ist.

7. Bedienungsvorrichtung (12) nach einem der Ansprüche 4-6, worin das verschiebbare Element (21) mit einem parallel zur Längsrichtung des verschiebbaren Elements (21) angeordneten Gestell (32) versehen ist, das während des Gebrauchs ein mit dem Antrieb (9) verbundenes Kettenrad (33) betätigt.

8. Bedienungsvorrichtung (12) nach einem der vorhergehenden Ansprüche, worin die Bedienungsvorrichtung (12) mit einem Zufuhrkanal (22, 23) versehen ist, der zum Zuführen einer Desinfektionsflüssigkeit in eine Öffnung im Instrument (11) während des Gebrauchs angeordnet ist.

9. Bedienungsvorrichtung (12) nach Anspruch 8, worin der Zufuhrkanal (22, 23) mit einer Druckanzeige verbunden ist.

10. Reinigungs- und Desinfektionsmaschine (1), die mit einem Desinfektionsraum (4) versehen ist, wobei ein Eingangsteil mit einer Seite des Desinfektionsraums (4) verbunden ist, um eine Bedienungsvorrichtung (12) nach einem der Ansprüche 1-9 mittels eines in der Maschine (1) angeordneten Transportsystems (6) dem Desinfektionsraum (4) zuzuführen, ein Ausgangsteil (3), das mit einer anderen Seite des Desinfektionsraums (4) verbunden ist, um die Bedienungsvorrichtung (12) aus dem Desinfektionsraum (4) mittels des Transportsystems (6) zu entfernen, worin an oder in der Nähe der Wand (15) des Desinfektionsraums (4) ein Antrieb zum Antreiben der Bedienungsvorrichtung (12), welche trennbar mit dem Antrieb (9) verbunden ist, während des Gebrauchs angeordnet ist, worin der Desinfektionsraum (4) während des Gebrauchs des Eingangsteils (2) sowie des Ausgangsteils (3) durch ein bewegliches Schließglied (7, 8) verschließbar ist.

11. Reinigungs- und Desinfektionsmaschine (1) nach Anspruch 10, die mit einem Erkennungssystem versehen ist, das angeordnet ist, um das Desinfektionsergebnis zu erkennen.

12. Reinigungs- und Desinfektionsmaschine (1) nach Anspruch 11, worin das Erkennungssystem in kommunikativer Verbindung mit einem Steuersystem steht, das so angeordnet ist, dass es dem Schließglied (8) ermöglicht, sich bei einem gewünschten Desinfektionsergebnis zu öffnen und die Bedienungsvorrichtung (12) durch das Transportsystem (6) zum Ausgangsteil (3) zu entfernen und dass das Schließglied (8) bei einem unerwünschten Desinfektionsergebnis geschlossen gehalten und eine weitere Desinfektion durchgeführt wird.

## Revendications

1. Dispositif d'actionnement (12) pour actionner un instrument médical (11) dans une machine de nettoyage et de désinfection (1), comprenant :
- une partie de montage (5) pour monter un instrument médical (11), où l'instrument (11) est pourvu d'un élément d'actionnement (24) avec lequel une partie d'instrument mobile (18) peut être déplacée,
- un organe d'activation (19 ; 19' ; 19"), lors de l'utilisation, prévu à proximité de l'élément d'actionnement (24), tel que sur la partie de montage (5), faisant face à l'instrument (11) et adapté pour s'engager avec l'élément d'actionnement (24),
- dans lequel l'organe d'activation (19 ; 19' ; 19") lors de l'utilisation est relié par entraînement à un dispositif d'entraînement (9), de sorte que la partie d'instrument mobile (18) puisse être déplacée par le dispositif d'entraînement (9),
- dans lequel deux organes d'activation ou plus sont prévus, agencés en une rangée (19, 19' ; 19', 19"), où le dispositif d'actionnement (12) comprend des moyens d'activation pouvant être entraînés par le dispositif d'entraînement (9) à proximité de la rangée d'organes d'activation (19, 19' ; 19', 19"), lesquels moyens sont agencés pour amener les organes d'activation (19, 19', 19") à être entraînés par le dispositif d'entraînement (9) de manière non simultanée.

2. Dispositif d'actionnement (12) selon la revendication 1, dans lequel un organe d'activation rotatif (19 ; 19' ; 19") comprend un évidement (20) agencé pour recevoir une came agencée sur l'élément d'actionnement rotatif (24) lors de l'utilisation.

3. Dispositif d'actionnement (12) selon la revendication 1 ou 2, dans lequel les organes d'activation agencés en une rangée (19, 19' ; 19', 19") sont agencés en rotation sur des arbres (25, 25', 25"), où les organes d'activation rotatifs (19, 19', 19") sont agencés pour, lors de l'utilisation, s'engager avec des éléments d'actionnement rotatifs (24) de l'instrument, où les axes de rotation sont essentiellement alignés, et chaque arbre (25 ; 25' ; 25") est pourvu d'une roue dentée (30 ; 30' ; 30"), qui peut être entraînée par les moyens d'activation.

4. Dispositif d'actionnement (12) selon la revendication 3, dans lequel les moyens d'activation comprennent un élément (21) qui peut être déplacé dans une direction longitudinale par rapport à la rangée de roues dentées (30, 30'), sur lequel élément une broche d'activation (26, 28) faisant face aux roues dentées (30, 30') est agencée, de sorte que lors du déplacement de la broche d'activation (26, 28) au-delà des roues dentées (30, 30'), elle s'engage avec les roues dentées (30, 30') de manière non simultanée.

5. Dispositif d'actionnement (12) selon la revendication 3, dans lequel les moyens d'activation comprennent un élément (21) avec deux broches d'activation ou plus (26, 27, 28) faisant face aux roues dentées respectives (30', 30"), lequel élément (21) peut être déplacé perpendiculairement à la direction de la rangée d'organes d'activation (19', 19"), où les broches d'activation sont agencées sur l'élément pouvant se déplacer dans la direction de déplacement en étant séparées d'une distance telle que lors du déplacement des broches d'activation (26, 27, 28) au-delà des roues dentées (30', 30"), ces broches s'engagent avec les roues dentées (30', 30") de manière non simultanée.

6. Dispositif d'actionnement (12) selon l'une des revendications 3 à 5, dans lequel un accouplement à glissement (31 ; 31' ; 31") est agencé entre l'arbre (25 ; 25' ; 25") et la roue dentée (30 ; 30'; 30").

7. Dispositif d'actionnement (12) selon l'une quelconque des revendications 4 à 6, dans lequel l'élément pouvant être déplacé (21) est pourvu d'une crémaillère (32) agencée parallèlement à la direction longitudinale de l'élément pouvant être déplacé (21), qui, lors de l'utilisation, s'engage avec une roue dentée (33) reliée au dispositif d'entraînement (9).

8. Dispositif d'actionnement (12) selon l'une des revendications précédentes, dans lequel le dispositif d'actionnement (12) est pourvu d'un conduit d'alimentation (22 ; 23) qui est agencé pour fournir, lors de l'utilisation, un fluide de désinfection dans une ouverture dans l'instrument (11).

9. Dispositif d'actionnement (12) selon la revendication 8, dans lequel le conduit d'alimentation (22 ; 23) est relié à un manomètre.

10. Machine de nettoyage et de désinfection (1) pourvue d'un espace de désinfection (4), d'une partie d'entrée reliée à un côté de l'espace de désinfection (4) pour fournir un dispositif d'actionnement (12) selon l'une des revendications 1 à 9 dans l'espace de désinfection (4) au moyen d'un système de transport (6) agencé dans la machine (1), d'une partie de sortie (3) reliée à un autre côté de l'espace de désinfection (4) pour retirer le dispositif d'actionnement (12) de l'espace de désinfection (4) par le système de transport (6), où sur ou à proximité d'une paroi (15) de l'espace de désinfection (4), un dispositif d'entraînement est agencé pour entraîner, lors de l'utilisation, le dispositif d'actionnement (12), qui est relié de manière amovible au dispositif d'entraînement (9), où l'espace de désinfection (4) lors de l'utilisation de la partie d'entrée (2) ainsi que de la partie de sortie (3) peut être fermé au moyen d'un organe de fermeture mobile (7, 8).

11. Machine de nettoyage et de désinfection (1) selon la revendication 10, pourvue d'un système de détection, qui est agencé pour détecter le résultat de désinfection.

12. Machine de nettoyage et de désinfection (1) selon la revendication 11, dans laquelle le système de détection est en liaison de communication avec un système de commande qui est agencé pour permettre à l'organe de fermeture (8) de s'ouvrir à un résultat de désinfection souhaité et pour permettre au dispositif d'actionnement (12) d'être retiré par le système de transport (6) vers la partie de sortie (3), et pour maintenir l'organe de fermeture (8) fermé à un résultat de désinfection non souhaité et effectuer une désinfection supplémentaire.
